# EUROPEAN PATENT SPECIFICATION

(11) **EP 1 434 533 B1**
(45) Date of publication and mention of the grant of the patent: **21.04.2010**
(21) Application number: 02778379.4
(22) Date of filing: 27.09.2002
(51) Int. Cl.: A61B 18/08, A61B 17/32

(54) **APPARATUS FOR MEASURING AND CONTROLLING THE EXPOSED LENGTH OF A TISSUE CUTTING DEVICE IN AN ENDOSCOPIC CATHETER**
GERÄT ZUR MESSUNG UND KONTROLLE DER FREILIEGENDEN LÄNGE EINER GEWEBESCHNEIDVORRICHTUNG IN EINEM ENDOSKOPISCHEN KATHETER
DISPOSITIF PERMETTANT DE MESURER ET DE REGLER LA PROFONDEUR DE LA LAME D'UN DISPOSITIF DE COUPE DE TISSUS DANS UN CATHETER ENDOSCOPIQUE

(30) Priority: 27.09.2001 US 963676
(43) Date of publication of application: 07.07.2004
(73) Proprietor: Boston Scientific Scimed, Inc., Maple Grove, MN 55311 (US)
(72) Inventor: CHIN, Yem, Burlington, MA 01803 (US); GRIEGO, John, Blackstone, MA 01504 (US)
(74) Representative: Altenburg, Udo
(86) International application number: PCT/US2002/030777
(87) International publication number: WO 2003/026524

(56) References cited:
- WO-A-01/67967
- US-A- 4 058 114
- US-A- 5 152 763
- US-A- 5 547 469
- US-A- 5 895 370

## Description

The present invention is an improvement of the devices disclosed in U.S. Patent No. 5,547,469, U.S. Patent No. 5,868,698 and U.S. Patent No. 5,683,362 and in U.S. Patent Application serial no. 09/154,834 in the name of Rowland, et al., all owned by the owner of the present patent.

### BACKGROUND

### 1. Field of the Invention

This invention generally relates to apparatus that is useful in performing diagnostic and therapeutic modalities in the biliary tree and more particularly to apparatus that is used in performing incisions within an endoscopic catheter for facilitating the diagnosis of gallstones in the bile duct and other portions of the biliary tree and the removal of such gallstones.

### 2. Description of Related Art

Historically the migration of gallstones into an individual's common bile duct was corrected by general surgical procedures. A surgeon would incise the bile duct and remove the gallstones and normally remove the gallbladder. In recent years less invasive treatment modalities have replaced these general surgical procedures and reduced patient trauma, long hospital stays and recovery periods.

For example, U.S. Pat. No. 4,696,668 and U.S. Pat. No. 4,781,677, both to Wilco, disclose a treatment modality involving the administration of a dissolution agent in the bile duct to essentially dissolve any gallstones. More specifically, a catheter contains several lumens for inflaming and deflating each of two balloons, venting bile, and infusing and aspirating the dissolution agent. Inflating the balloons occludes the bile duct at two spaced sites and creates a sealed spaced that receives the dissolution agent. As the space is sealed from the remaining biliary tree, the dissolution agent finds access to the gallbladder and any gallstones therein through the cystic duct with the exclusion of bile from the gallbladder fundus. The dissolution agent also will be confined in high concentration around bile duct gallstones. After the gallstones dissolve the balloons are deflated and the catheter can be withdrawn. In this particular approach, the catheter is directed into the biliary tree using a standard duodenoscope that passes through the alimentary tract. Although this and analogous approaches have the potential of minimizing patient trauma, such treatments require extended placement of the duodenoscope in the patient, exhibit low efficacy and introduce a potential for adverse reactions to the dissolution agents.

In an alternative approach, a surgeon directs a surgical extractor into the biliary tree through at least an incision in the bile duct. For example, in U.S. Pat. No. 3,108,593 to Glassman a surgeon incises both the bile duct and duodenum. Then the surgeon directs an extractor through the bile duct incision, biliary tree, sphincter of Oddi and duodenum to exit through the duodenum incision. This extractor includes a series of longitudinally spaced cages for trapping any gallstones in the bile duct and removing them through either of the incisions.

U.S. Pat. No. 4,627,837 to Gonzalo discloses a catheter device with a pair of inflatable balloons at its distal end. This catheter is led through an incision in the bile duct toward the duodenum. After the distal balloon passes through the sphincter of Oddi, both balloons are expanded to anchor the catheter in place. This enables the catheter to be used for irrigating and flushing through other lumens in order to capture any gallstone in the second balloon for removal through the incised bile duct.

In accordance with still another modality as for the treatment of strictures, a surgeon may insert a catheter device through the bile duct or duodenum for the purpose of dilating or enlarging the sphincter of Oddi. For example, U.S. Pat. No. 4,705,041 to Kim discloses a dilator that is directed through an incision in the bile duct and the sphincter of Oddi. An expandable tip dilates the sphincter of Oddi. U.S. Pat. No. 5,035,696 to Rydell discloses an electrosurgical instrument that is directed through the duodenum and to the sphincter of Oddi for performing a sphincterotomy. This apparatus contains a cutting wire that is heated to cut the sphincter muscle. U.S. Pat. No. 5,024,617 to Karpiel, discloses a similar device that can be directed through a duodenoscope. U.S. Pat. No. 5,152,771 to Swell, Jr. discloses a device for performing a sphincterotomy that is directed through an incision in the bile duct and includes a knife for cutting the sphincter muscle.

WO-A1-01/67967 discloses a surgical snare device for ensnaring objects in the vascular system, the snare including: a core wire with a proximal end and a distal end; a tubing that extends over the core wire, whereby the tubing includes a metal section; and a loop with a distal end and a proximal end, whereby the loop attaches at the proximal end to the distal end of the core wire. The tubing includes a radiopaque marker at its distal end, and the loop includes a radiopaque collar at its distal end to provide visibility.

The use of the duodenoscope and sphincterotomy device, such as shown in the Rydell and Karpiel patents, enables an internist to diagnose and treat problems in the biliary tree with minimal patient invasion. For example, modalities as described in these patents eliminates the surgery needed for incising the bile duct. Consequently, these modalities can be performed as outpatient or day surgical procedures. These procedures greatly reduce patient trauma, the length of a hospital stay and recovery times. For example, if an internist determines that gallstones are present in the biliary tree, particularly the common bile duct, the internist can insert a duodenoscope into the duodenum to view the sphincter of Oddi. Then a first catheter can be advanced through the working channel of the duodenoscope with or without a guidewire and directed through the sphincter of Oddi into the biliary tree. Contrast agent injected through the catheter enables fluoroscopy or other imaging procedures to confirm the presence of gallstones within the biliary tree. Next the internist exchanges the first catheter for a second catheter for performing a sphincterotomy such as the type disclosed in the above-identified Rydell and Karpiel patents. The second catheter is then exchanged for a third catheter such as shown in the Glassman patent or some other equivalent retrieval catheter for drawings gallstones through the enlarged sphincter of Oddi. Thereafter the retrieval catheter is manipulated to release the gallstone into the duodenum. The catheter, any guidewire and the duodenoscope can then be removed to complete the procedure.

This procedure is significantly less traumatic to the patient than other prior art procedures because the only incision occurs during the sphincterotomy. However, this procedure, as described above, requires three separate catheters and two catheter exchanges. These exchanges are required because the first, second and third catheters function solely to inject contrast agent to perform the sphincterotomy and to dislodge gallstones, respectively. The time required for performing each catheter exchange can increase patient trauma and increase the duration of the procedures and reduce efficiency. Moreover, each such procedure requires the use of two or three separate catheter devices.

Multi-lumen catheters are available which typically reduce the number of catheters and catheter exchanges used during a procedure and thereby reduce both the time required and the patient's trauma while increase efficiency. The use of multi-lumen devices also eliminates the need for the repositioning of subsequent catheters because the original catheter was withdrawn. While the multi-lumen device may have to be repositioned, the repositioning is considerable less then when a single lumen catheter is used. While precision positioning of the multi-lumen device is essential for safe and effective results, accurate positioning of the multi-lumen device is difficult to achieve. State of the art multi-lumen devices are typically positioned by torque transmission from the handle to the distal tip approximately 6 feet away. Additionally, when an incision is made, proper knife depth is difficult to maintain because of the connection between the knife lumen and the knife shaft. When pressure is applied to the knife lumen an undesirable movement of the needle knife tip may occur because of this imprecise connection.

A need exists for an apparatus of accurate placement of catheters, multi-lumen devices and needle knives. A further need exists for an apparatus for an accurate depth control for needle knives and other cutting instruments.

### SUMMARY

The above mentioned problems are solved according to the invention by a catheter as defined in claim 1. Advantageous embodiments are defined in the dependent claims.

Therefore, this invention provides an apparatus for accurate depth control of incisions performed through an endoscopic catheter. The invention may also provide an apparatus for resisting pressures felt on the cutting instrument which tend to push the cutting instrument back towards the lumen.

The invention is an endoscopic catheter which has a distally located tissue cutting device in a lumen thereof comprising an exposed linear cutting member, the improvement for determining the amount of cutting member deployed for cutting which comprises providing the cutting member with a plurality of radiopaque indicia located at radiologically measurable intervals. According to the invention a radiopaque reference point is included which can be used to determine the length of the deployed cutting member by reference to the indicia. The cutting member may be a needle knife where the reference point is at the distal end of the catheter or a phincterotome where the reference point is on the catheter proximal to the cutting member of the sphincterotome. The radiopaque indicia can be referenced from the middle of the cutting member and may include markings along the length of the cutting member as a function of the distance from the middle.

### BRIEF DESCRIPTION OF THE DRAWINGS

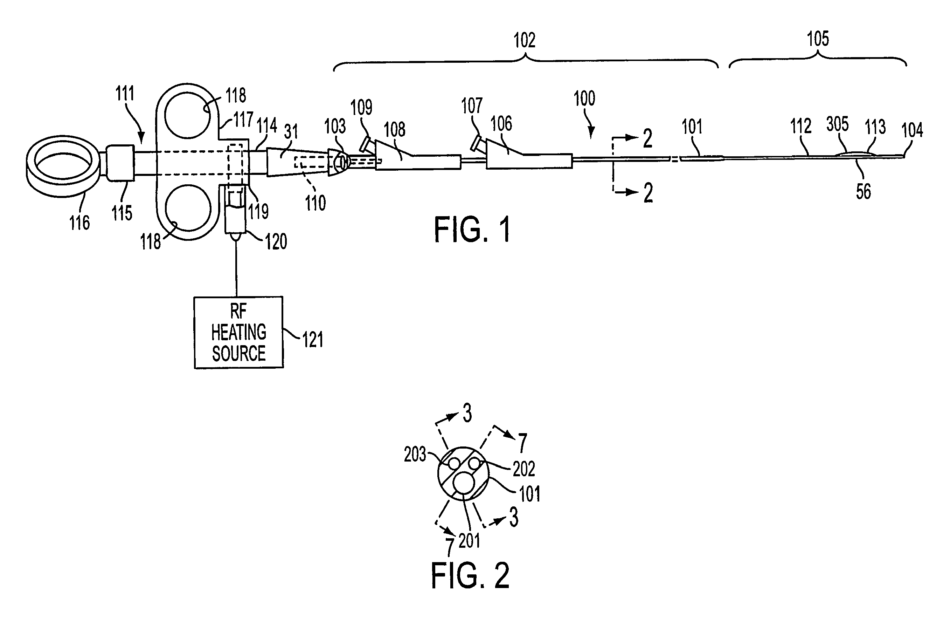

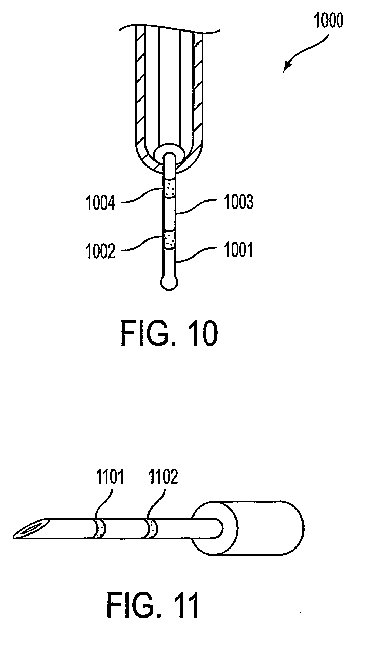

The various objects, advantages and novel features of this invention will be more fully apparent from a reading of the following detailed description in conjunction with the accompanying drawings in which like reference numerals refer to like parts, and in which only the apparatuses shown in Figs. 10-12 are embodiments of the present invention, whereas the apparatuses shown in Figs. 1-9 do not fall under the scope of the claims:
FIG. 1 is a plan view of a catheter apparatus;
FIG. 2 is a cross-section taken along lines 2--2 in FIG. 1;
FIG. 3 is a cross-section taken along lines 3-3 in FIG. 2;
FIG. 4 depicts the apparatus of FIG. 1 positioned through a duodenoscope for injecting contrast agent into the biliary tree.
FIG. 5 is an enlarged view that depicts the orientation of the apparatus in FIG. 1 for performing a sphincterotomy;
FIG. 6 depicts the apparatus of FIG. 1 positioned through a duodenoscope for dislodging material within the common bile duct;
FIG. 7 is a cross-section of an alternative of the apparatus as viewed generally along lines 7-7 in FIG. 2;
FIG. 8 is a cross-section of still another alternative taken along lines 7-7 in FIG. 2;
FIG. 9 is an enlarged diagram of an apparatus for preventing movement in the cutting member once the user has adjusted the cutting member to the length desired;
FIGURE 10 is a prospective view of a blade marking to allow the user to position the needle knife to the correct length;
FIGURE 11 is an alternate embodiment of the blade marking of the current invention; and
FIGURE 12 is a prospective view indicating how the markings would be applied to a cutting wire or member.

### DESCRIPTION OF ILLUSTRATED EMBODIMENTS

FIG. 1 depicts catheter apparatus 100 that has the capability of injecting a contrast age into the biliary tree, accurately positioning a cutting wire, of performing a sphincterotomy and of dislodging a gallstone into the duodenum. Apparatus 100 includes a catheter 101 which, for purposes of definition, includes proximal portion 102 extending from proximal end 103 and distal end 104 with distal portion 105 extending a short distance from distal end 104. In a typical application, the catheter will have a working length of 200 cm and distal portion 105 will have a length of 6 cm to 9 cm. Normally distal portion 105 will have a diameter that is smaller than the diameter of proximal portion 102 to increase the flexibility of distal portion 105. The reduction in diameter also makes distal end 104 less traumatic and allows distal portion 105 to reach smaller passages while allowing the larger proximal portion 102 to provide necessary hoop strength and rigidity, particularly where proximal portion 102 is coextensive with the working channel of a duodenoscope. For example, the proximal and distal portions might have diameters corresponding to 7 Fr and 5.5 Fr catheter sizes (*i.e.,* 0.09" and 0.07" respectively).

As shown particularly in FIG. 2, catheter 101 has three lumens. First lumen 201 has a diameter that is greater than either second lumen 203 or third lumen 203. In one particular example first lumen 201 has a diameter of 0.040" in proximal portion 102 that reduces to about 0.037" in distal portion 105 to receive a standard 0.035" guidewire. In addition, first lumen 201 is offset from the center of the catheter 101.

The cross section of both second lumen 202 and third lumen 203 are each smaller than the cross section of first lumen 201 and are radially offset from the centerline of catheter 101, from each other and from first lumen 201. In one particular example the cross section of third lumen 203 has a diameter of 0.028" in proximal portion 102 that reduces to about 0.020" in distal portion 105 and second lumen 202 has an internal diameter of 0.028" in proximal portion 102 that reduces to about 0.020" in distal portion 105. As described later, this third lumen 203 carries a cutting wire for performing a sphincterotomy and for allowing the infusion of a contrast agent at reasonable rates. The cutting wire can also be positioned, as described later, as desired. The angular spacing between second lumen 202 and third lumen 203 is about 45 degrees and the angular spacing between first lumen 201 and each of lumens 202 and 203 each is about 157.5 degrees. In this configuration and with these dimensions proximal portion 102 readily passes through the working channel of any duodenoscope.

Referring again to FIGS. 1 and 2, each of lumens 201, 202 and 203 includes an entry port in proximal portion 102 and an exit port in distal portion 105. Generally, and as described in more detail later, first lumen 201 has an exit port through distal end 104 while the exit ports for lumens 202 and 203 can be sited at different locations in distal portion 105 depending upon a particular application.

In FIG. 1, the entry ports in proximal portion 102 adjacent proximal end 103 include an entry port 106 that provides access to first lumen 201 and includes an optional Leur lock fitting 107. Proximally positioned entry port 108 provides access to second lumen 202 and includes optional Leur lock fitting 109. Proximal entry port 110 for third lumen 203 is located coextensively with a portion of handle 111 attached to proximal end 103. One of ordinary skill in the art would understand that this specific configuration is given as an example.

Referring to the distal portion 105, catheter 101 in this particular example carries expansible balloon 112 proximally of the excursion of cutting wire 113 externally of catheter 101. As described in U.S. Patent Application serial no. 09/154,834 in the name of Rowland, *et al*., and owned by the owner of the present application and already incorporated herein by reference in its entirety, second lumen 202 emerges at a distal exit port through the side of catheter 101 with the interior of expansible balloon 112. An extension of second lumen 202 beyond the distal port is sealed by known methods of manufacture. Consequently, fluid forced through entrance port 108, as by a syringe (not shown) attached to Leur lock fitting 109, expands balloon 112 into an occluding orientation with an inflated diameter in the range up to 20 mm.

First lumen 201 extends through catheter 101 and terminates with an exit port in distal end 104. Thus first lumen 201 is adapted for receiving a guidewire through the entry port 106 that will extend through catheter 101 and exit distal end 104 and allow the catheter to slide over that guidewire.

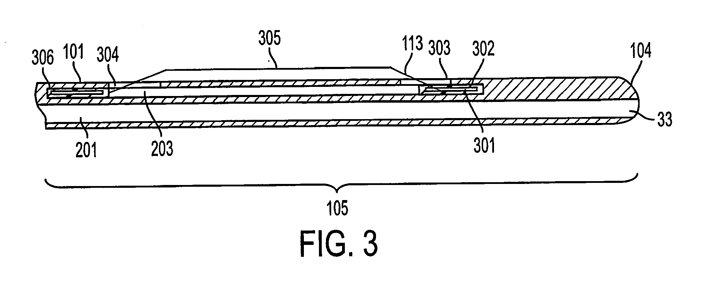

Referring to FIG. 3, distal end 301 of cutting wire 113 attaches to a clamp 302 formed at the distal end of third lumen 203. Spaced skived ports 303 and 304 allow active portion 305 of the cutting wire 113 to emerge from catheter 101 through skived aperture 303, parallel the catheter 101 exteriorly thereof and return into third lumen 203 through port 304 and reinforcing sleeve 306. Cutting wire 113 then extends through third lumen 203 to handle 111 shown in FIG. 1 where it emerges as proximal end portion 114.

Handle 111, as shown in FIG. 1, includes central member 115 terminating with thumb ring 116. The central member 115 extends through and slides with respect to body section 117 having opposed finger rings 118. The central member 115 also attaches to catheter 101, and is therefore an extension of catheter 101. Member 117 additionally includes internal connector 119 for clamping proximal end 114 of cutting wire 113. Thus, when body 117 is at its distal position as shown in FIG. 1, distal portion 105 of catheter 101 is in essentially straight line as shown in FIG. 1 with active portion 305 of cutting wire 113 being closely adjacent catheter 101. Retracting body portion 117, causes cutting wire 113 to bend distal end 104 upwardly as shown in FIG. 3 to a position that is essentially at right angles to the main axis of the catheter, as will be shown later.

Connector block 119 and cutting wire 113 are generally conductive members that attach through RF connector 120 to RF heating source 121. The use of such RF heating sources 121 for energizing cutting wire 113 thereby to cut the sphincter muscle is well known in the art and represents one possible sphincterotomy procedure that can be adapted and is not described further.

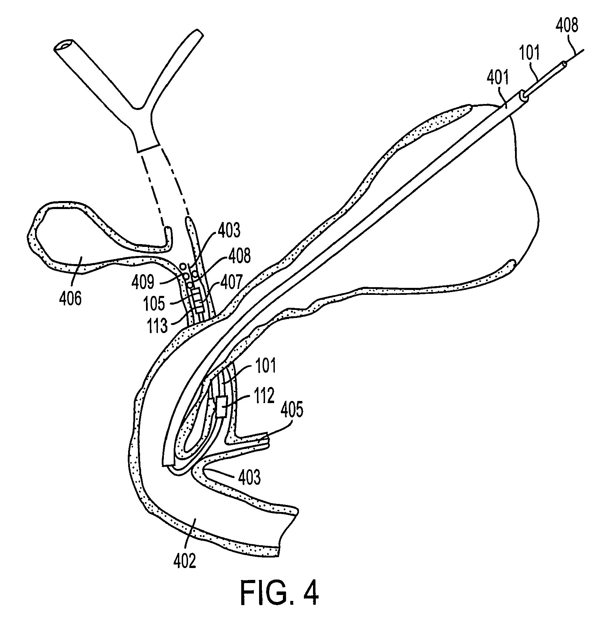

With this description of the apparatus structure, it will now be possible to understand its use in a particular application. FIG. 4 discloses, in a partially broken and schematic view, the positioning of duodenoscope 401 in duodenum 402 adjacent sphincter of Oddi 403. Catheter 101 such as constructed in FIG. 1 passes through sphincter of Oddi 403 into the common bile duct 404, bypassing pancreatic duct 405. Distal end 104 does not extend to gallbladder 406.

Fluoroscopy allows the appropriate positioning by utilizing a series of radio-opaque markers 406 at distal portion 105 that may include clamp 302 and reinforcing sleeve 306 in FIG. 3. Catheter 101 can be positioned with or without the presence of guidewire 408 in first lumen 201 shown in FIGS. 2, and 3. For purposes of injecting the contrast agent, any guidewire 408 can be withdrawn to allow the contrast agent to be injected through first lumen 201 for purposes of fluoroscopic examination to confirm the presence of one or more gallstones 409. It is also possible during the operation to expand balloon 112 to occlude common bile duct 404 and block any migration of contrast agent into duodenum 402 or pancreatic duct 405.

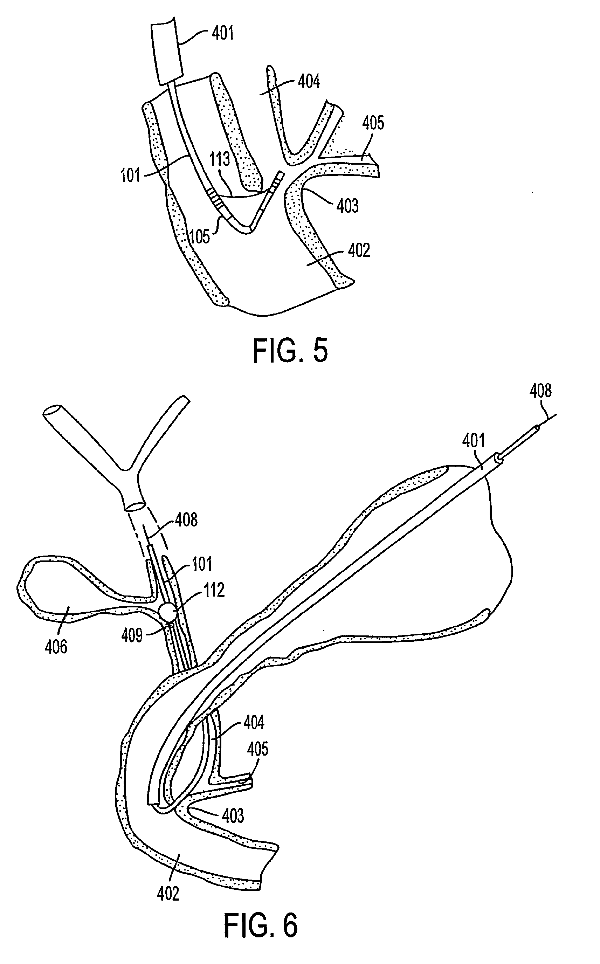

FIG. 5 is an enlarged view showing duodenum 402, sphincter of Oddi 403, portions of pancreatic duct 405 and common bile duct 404. In FIG. 5 catheter 101 has been positioned relative to the duodenoscope 401 through the opening of sphincter of Oddi 403. The handle 111 in FIG. 1 has been drawn proximally to deflect distal portion 105 into essentially a right angle configuration such that cutting wire 113 abuts a portion of sphincter of Oddi 403. The application of RF heating to cutting wire 113 then will cut sphincter of Oddi 403 and enlarge the opening therethrough. As will be apparent, the sphincterotomy is performed with direct visualization of the sphincter of Oddi through the duodenoscope.

Moreover, as has been observed by others, catheters having guidewire and cutting wire lumens tend to assume a particular angular orientation when distal portion 105 emerges from the duodenoscope. This orientation is essentially independent of the angular position of the catheter when it is inserted into the duodenoscope. The offset nature of lumen 203 as shown in FIG. 2, improves the location of cutting wire 113 as distal portion 105 passes through sphincter of Oddi 403. Specifically the angularly offset brings cutting wire 113 into better alignment with common bile duct 404 and displaces the cutting wire from pancreatic duct 405.

FIG. 6 depicts the catheter after the sphincterotomy and after catheter 101 is advanced over guidewire 408, if used. FIG. 6 also discloses catheter 101 after balloon 112 has been moved beyond gallstone 409 in bile duct 404. Balloon 112 is expanded so that upon withdrawal of catheter 101 balloon 112 will dislodge gallstones 409 and sweep them through sphincter of Oddi 403 into duodenum 402.

As will now be apparent from the description of the particular catheter apparatus 100 shown in FIG. 1 and its use as discussed with respect to FIGS. 4, 5, and 6, the single catheter apparatus is capable of providing diagnostic contrast agent injection, of performing a sphincterotomy and of dislodging gallstones in the common bile duct or other portions of the biliary tree without having to exchange a catheter. Moreover, positioning and sizing of the lumens enables these functions to be performed with a catheter apparatus that is readily adapted for use in the working channels of standard duodenoscopes. Consequently the gallstones can be removed from the biliary tree without bile duct incisions and accompanying surgical procedures, as duodenoscope can be introduced through the alimentary tract. Consequently the entire procedure is adapted for being performed more rapidly than prior art procedures and with fewer components. The net effect is to reduce patient trauma and the overall time and cost of conducting the procedure.

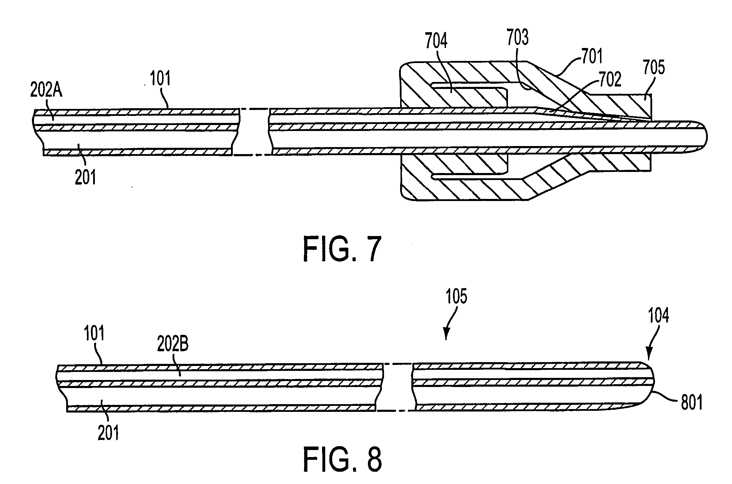

In FIG. 1 balloon 112 is located proximally of cutting wire 113. FIG. 7 discloses an alternative in which balloon 701 is located distally of cutting wire 113. More specifically, the distal end of lumen 202A, corresponding to second lumen 202 in FIG. 3 is sealed. Side facing exit port 702 skived or otherwise formed in catheter 101 opens into chamber 703 formed by balloon 701. First sealing portion 704 and a sealing portion 705 of balloon 701 connect proximally and distally of aperture 702 respectively and seal chamber 703.

Introduction of a balloon inflation fluid through lumen 202A expands balloon 701 into an occluding orientation corresponding to the orientation of balloon 701. Retraction of catheter 101 with distal balloon 701 inflated enables withdrawal of a gallstone from the bile duct. This particular example is particularly adapted when it is determined that a gallstone is located high in the biliary tree to minimize the incursion of distal portion 105 through the biliary tree beyond the gallstone or in any application in which the internist desires to minimize the length of distal portion 105 that extends beyond the occluding balloon.

FIG. 8 discloses another alternative for enlarging the sphincter of Oddi and performing another procedure, such as injecting a contrast agent into the biliary tree, as might be used in the diagnosis and treatment of a stricture in the biliary tree. In this particular example exit port 801 from second lumen 202B is located in distal end 104 of distal portion 105. First lumen 201 then can be used for a guidewire and lumen 202B, for injecting the contrast agent directly into the biliary tree while the guidewire remains in place. The apparatus would then be positioned to perform a sphincterotomy without having to exchange a catheter should the procedure be warranted.

As still another alternative, the internist could utilize a conventional catheter for purposes of injecting the contrast agent to determine the need for gallstone removal. If treatment were indicated, the internist could then utilize apparatus as shown in FIG. 1 with a single exchange over the guidewire that would pass through lumen 201 as previously described.

As can be seen from the above description one of the steps in the treatment of obstructive disease is normally the practice of tissue incision which is achieved by advancing a cutting wire endoscopically to the target site. As explained above, once the catheter tip is in position, the catheter tip is bowed (FIG. 5) to expose cutting wire 113 to tissue. Diathermic current is then passed through cutting wire 113 from RF Heating Source 121 (FIG. 1) which allows the endoscopist to incise and cauterize the tissue at the target site. Safe and effective results are only obtained through precision positioning of cutting wire 113 and control of the portion of the exposed cutting wire. Similarly, when a needle knife distally extends from the end of a catheter, the portion of the needle knife exposed must also be accurately known and maintained throughout the cutting procedure.

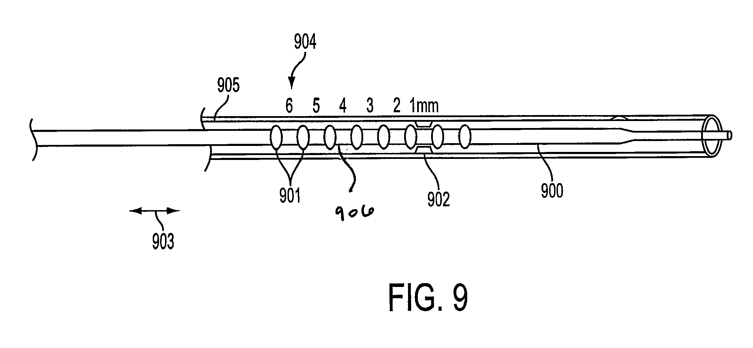

FIGURE 9 is an enlarged diagram of an apparatus for preventing movement of the cutting member once the user has adjusted the cutting member to the length desired. On the cutting member 900, a series of detents, pawls, or raised areas 901 are placed. Between these raised areas 901 are lower areas or indentations 906. The indentations 906 interact with a corresponding raised area 902 which is located along the inner circumferences of the lumen 905. The interactions between the corresponding raised area 902 provides resistance to movement in the cutting member in the direction of 903 when the cutting member is used. One of ordinary skill in the art would understand that detents includes beads, bumps, and similar surface features and their shapes could be round, pointed, sloped. The movement which is being opposed is an axial shifting of the cutting member, (wire), in either direction, within the lumen. One of ordinary skill would also understand that the placement of the detents, pawls, or raised areas 901 and corresponding raised areas 902 could be reversed. The spacing between the raised areas may also be used as an indication to the length 904 of cutting member 900 which is exposed.

FIGURE 10 is a prospective view of a blade marking 1000 methodology to allow the user to position the needle knife to the correct length. According to the invention, the blade marking are radiopaque indicia which may be color markings, various coatings (anodized, acid dipped), inked, etched or similar techniques which would allow the user to radiologically determine the length of the exposed needle knife. The markings may indicate fixed distances such as 1 mm. For example in FIGURE 10, Sections 1001, 1002, 1003, and 1004 may each be 1 mm in length.

FIGURE 11 is an alternate embodiment of the present invention in which lines 1101, 1102 etched, marked, or written on the needle knife indicate the various lengths of the exposed blade. One of ordinary skill in the art would understand that any type of markings which allowed the user to radiologically determine the length of the exposed blade would fall within the disclosed invention.

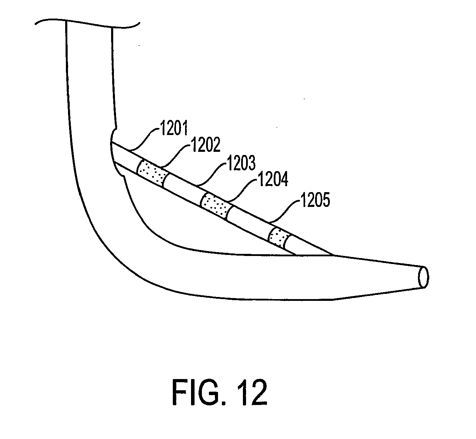

FIGURE 12 is a prospective view indicating how the markings would be applied to a cutting wire 1201. Sections of the cutting wire 1201 may be alternately marked 1202, 1203, 1204, 1205 to indicate fixed lengths of the exposed wire.

If an endoscopy procedures were performed under direct visualization, the markings illustrated in FIGURES 10, 11, and 12 would allow the user to directly visualize the length of the exposed cutting wire or knife. Once the user has adjusted the needle knife or cutting wire to the desired length, the exposed blade must be fixed. In order to fix the length of the exposed blade, the cutting wire must be held at two different places. First, the sliding handle with a locking mechanism on the proximal end must be locked. This first locking mechanism has been described with respect to FIGURE 1 and optional Leur lock fitting 107 and optional Leur lock fitting 109. Once the sliding handle has been locked using a Leur lock fitting (or similar device) the distal end of the cutting wire must also be held to ensure the exposed knife is not pushed back into the lumen.

For endoscopy procedures which are performed using x-rays, radiopaque indicia, markings, colors, numbers, letters, etc. are included on the cutting instrument to indicate the length of the exposed cutting surface. A reference point is also included to indicate the beginning of the exposed cutting surface and a comparison between the reference point and the radiopaque indicia will allow the user to determine the exposed cutting surface.

## Claims

1. Endoscopic catheter (100) with a distally located tissue cutting device in a lumen thereof comprising an exposed linear cutting member (900),
wherein
the linear cutting member (900) is improved for determining the amount of cutting member (900) deployed for cutting by providing said cutting member (900)
with a plurality of radiopaque indicia located at radiologically measurable intervals, and said endoscopic catheter (100) has a radiopaque reference point to determine the length of the deployed cutting member (900) by reference to said radiopaque indicia.

2. Catheter of claim 2 wherein the cutting member (900) is a needle knife and said radiopaque reference point is at the distal end (104) of said catheter (100).

3. Catheter of claim 1 wherein the cutting member (900) is a sphincterotome wire and said radiopaque reference point is on said catheter (100) proximal to said wire.

4. Catheter of claim 1 wherein said radiopaque indicia are referenced from a middle of said cutting member (900) and alternate along a length of said cutting member (900) as a function of the distance from said middle thereof.

## Patentansprüche

1. Endoskopischer Katheter (100) mit einer distal angeordneten Gewebeschneidvorrichtung in einem Lumen davon, ein freiliegendes lineares Schneidbauteil (900) umfassend,
wobei das lineare Schneidbauteil (900) zur Bestimmung der Menge des Schneidbauteils (900) verbessert ist, welches zum Schneiden ausgefahren ist, durch Bereitstellen des Schneidbauteils (900) mit einer Vielzahl von radioopaken Indikatoren, die in radiologisch messbaren Intervallen angeordnet sind, und
wobei der endoskopische Katheter (100) einen radioopaken Referenzpunkt aufweist, zur Bestimmung der Länge des ausgefahrenen Schneidbauteils (900) durch Bezug auf die radioopaken Indikatoren.

2. Katheter nach Anspruch 2, wobei das Schneidbauteil (900) ein Nadelmesser ist, und der radioopake Referenzpunkt an dem distalen Ende (104) des Katheters (100) angeordnet ist.

3. Katheter nach Anspruch 1, wobei das Schneidbauteil (900) ein Sphinkterotomdraht ist, und der radioopake Referenzpunkt auf dem Katheter (100) proximal zu dem Draht angeordnet ist.

4. Katheter nach Anspruch 1, wobei die radioopaken Indikatoren von einer Mitte des Schneidbauteils (900) referenziert sind, und sich entlang einer Länge des Schneidbauteils (900) als Funktion der Entfernung von der Mitte davon ändern.

## Revendications

1. Cathéter endoscopique (100) avec un dispositif de découpe de tissus situé distalement dans une lumière de celui-ci comprenant un organe de découpe linéaire exposé (900), dans lequel l'organe de découpe linéaire (900) est perfectionné pour déterminer la quantité de l'organe de découpe (900) déployée pour la découpe en pourvoyant ledit organe de découpe (900) d'une pluralité d'index radio-opaques situés à intervalles radiologiquement mesurables, et ledit cathéter endoscopique (100) possède un point de référence radio-opaque pour déterminer la longueur de l'organe de découpe (900) déployé par référence auxdits index.

2. Le cathéter de la revendication 1, dans lequel l'organe de découpe (900) est un couteau en aiguille et ledit point de référence radio-opaque est à l'extrémité distale dudit cathéter (100).

3. Le cathéter de la revendication 1, dans lequel l'organe de découpe (900) est un fil de sphincterotome et ledit point de référence radio-opaque est sur ledit cathéter (100) à proximité dudit fil.

4. Le cathéter de la revendication 1, dans lequel lesdits indices de référence radio-opaques sont référencés à partir d'un milieu dudit organe de découpe (900) et alternent le long d'une longueur dudit organe de découpe (900) en fonction de la distance depuis ledit milieu de celui-ci.
